# EUROPEAN PATENT APPLICATION

(11) **EP 2 615 176 A1**
(43) Date of publication of application: **17.07.2013**
(21) Application number: 12000142.5
(22) Date of filing: 11.01.2012
(51) Int. Cl.: C12N 15/867, C07K 14/12, C07K 16/24, C07K 16/28, C12N 5/0735, C12N 5/0797

(54) **Novel pseudotyped lentiviral particles and their use in the in vitro targeted transduction of undifferentiated pluripotent human embryonic stem cells and induced pluripotent stem cells**

(71) Applicant: Paul-Ehrlich-Institut Bundesamt für Sera und Impfstoffe, 63225 Langen (DE)
(72) Inventor: Schneider, Irene, 63456 Hanau (DE); Buchholz, Christian, 60594 Frankfurt (DE); Schumann, Gerald, 63303 Dreieich (DE); Klawitter, Sabine, 67433 Neustadt/Weinstrasse (DE)
(74) Representative: Grund, Martin

(57) **Abstract**

The inventors developed novel pseudotyped lentiviral vector particles comprising a morbillivirus fusion (F) protein and a mutated hemagglutinin (H) protein of the measles virus (MeV) or the Edmonton strain of the measles virus (MeV_{Edm}), wherein the cytoplasmic portions of the F and the H protein are truncated, and wherein the amino acids necessary for receptor recognition in the H protein are mutated that it does not interact with CD46, SLAM and/or nectin-4 and further has a single chain antibody to a cell surface marker of hESCs and iPSCs at its ectodomain. In this invention, the single chain variable fragment (scFv) anti-cell surface marker coding sequence of the single chain antibody is fused to the coding sequence at the ectodomain of the H protein, wherein the single chain antibody is selected from the group consisting of CD30, EpCAM (CD326), CD9, Thy-1 (CD90), SSEA-3, SSEA-4, TRA-1-60 or TRA-1-81. The transduction according to the present invention does not interfere with the pluripotency, i.e. present transduced hESCs and iPSCs remain undifferentiated, i.e. are able differentiate into all germ layer lineages.

## Description

### Technical Field

The present invention relates to novel pseudotyped lentiviral particles and to their use in the *in vitro* targeted transduction of undifferentiated pluripotent human embryonic stem cells (hESCs) and induced pluripotent stem cells (iPSCs).

### Prior art

Stem cells are undifferentiated cells found in all multicellular organisms that can divide through mitosis and differentiate into diverse specialized cell types and can self renew to produce more stem cells. They are able to differentiate into a variety of cell types and, accordingly, may be a source of replacement cells and tissues that are damaged in the course of disease, infection, or because of congenital abnormalities (Lovell-Badge, R., Nature, 2001, 414: 88-91). Various types of stem cells exist that are characterized by their differentiation potential *in vivo* and *in vitro,* namely depending on the tissue they are isolated from. They carry out the unique functions of particular tissues when they differentiate in mature cells, wherein pluripotent stem cells are thought to have the potential to differentiate in almost any cell type, while multipotent stem cells are believed to have the potential to differentiate into many cell types only.

Pluripotent stem cells (PSCs) are available for example as undifferentiated embryonic stem cells (ESCs) and can be cultured *in vitro* as permanent lines without undergoing differentiation. They are able to generate all cell types of the body, but not the umbilical cord, trophoblasts and associated structures. Although they are unable to generate a functional organism, they provide the opportunity to develop stem cell-based therapies for human diseases and tissue repair (Leeb, C. et al., Cell Prolif., 2011, 44, Suppl. 1: 9-14). The first successful derivation of human ESCs (hESCs) has been reported in 1998 (Thomson, J. A. et al., Science, 1998, 282: 1145-1147), where the hESCs are derived from the inner cell mass of a blastocyst before it would have implanted in the uterine wall. Gearhart and co-workers derived human embryonic germ (hEGC) cell lines from fetal gonadal tissue (US 6,090,622). Both hESCs and hEGCs have the long-sought characteristics of pluripotent stem cells: they can be cultured extensively without differentiating, they have a normal karyotype, and they are capable of producing a number of important cell types. A significant challenge to the use of pluripotent stem cells for therapy is that they are traditionally cultured on a layer of feeder cells to prevent differentiation (US 5,843,780; US 6,090,622). According to Thomson et al., (supra), hESCs cultures without feeders soon die, or differentiate into a heterogeneous population of committed cells.

Because the aforementioned methods require the destruction of early embryos, alternative sources for hESCs have been exploited for ethical reasons. An alternative pluripotent stem cell type of significance has been introduced in 2006 where *in vitro* cultured somatic cells have been reversed into an embryonic-like pluripotent state leading to the formation of induced pluripotent stem cells (iPSCs; Takahashi, K. & S. Yamanaka, Cell, 2006, 126: 663-676). The reprogramming is performed by introduction of certain stem cell-associated genes, i.e. of 3-5 factors (Krüppel-like factor 4, Klf-4; sex determining region Y-box, Sox, e.g. Sox-2; octamer binding transcription factor 4, Oct4; transcription factor Nanog; transcription factor cMyc) into non-pluripotent, i.e. differentiated cells (e.g. fibroblasts) either by viral transduction or transfection. This reprogramming technology avoids all ethical problems with hESCs and even more important, enables the generation of pluripotent cells for autologous cell therapies as iPSC can be derived directly from the patient for personalised medicine approaches. Furthermore it allows the application of human iPSCs to specify issues in drug research and in safety pharmacological or toxicological studies (Leeb, C. et al., Cell Prolif., 2011, 44, Suppl. 1: 9-14).

So far, many promising studies have shown the therapeutic and scientific potential of stem cells, in particular of hESCs and iPSCs. Progress in hESC and iPSC application was achieved by improving techniques for the gene transfer. Stably transfected stem cells can be used for example as cellular vehicle for protein-supplement gene therapy and/or to direct the stem cells into particular lineages. Furthermore, insertion of lineage-specific reporter constructs would allow isolation of lineage-specific cells, and would allow drug discovery, target validation, and/or stem cell based studies of gene function and the like. However, current strategies of gene transfer do not distinguish between cells of a stem cell phenotype and cells that entered in a differentiation pathway or feeder cells. Additionally, current methods are usually very inefficient and often toxic to the iPSCs, since the cells have to be trypsinised and separated from feeder cells to allow transduction with sufficient efficiency (Jang, J. E. et al., Stem Cells Dev., 2006, 15: 109-17). Only a minor fraction of the cells survives this procedure. A method using adenovirus vector mediated transduction only allows efficient gene transfer when the cells are cultivated in a special medium supplemented with a Rho-kinase inhibitor (Tashiro, K. et al., Cell Reprogramming, 2010, 12: 501-507). Thus, current methodology depends on manipulations of the cells before transduction, which are complex and can reduce the stem cell characteristics of the cells.

### Problem of the invention

Thus, the problem of the present invention is to provide means and/or a method how to achieve targeted gene transfer into undifferentiated human embryonic stem cells and induced pluripotent stem cells, which avoid the disadvantages as mentioned above and are superior with respect to the specificity and efficiency of transduction.

### Summary of the invention

The technical problem is solved by the subject-matter of claims 1, 12, 14 and 15. Preferred embodiments are the subject-matter of the dependent claims.

The inventors developed novel pseudotyped lentiviral vector particles comprising a morbillivirus fusion (F) protein and a mutated hemagglutinin (H) protein of the measles virus (MeV) or the Edmonton strain of the measles virus (MeV_{Edm}), wherein the cytoplasmic portions of the F and the H protein are truncated, and wherein the amino acids necessary for receptor recognition in the H protein are mutated that it does not interact with CD46, SLAM and/or nectin-4 and further has a single chain antibody to a cell surface marker of hESCs and iPSCs at its ectodomain. In this invention, the single chain variable fragment (scFv) anti-cell surface marker coding sequence of the single chain antibody is fused to the coding sequence at the ectodomain of the H protein, wherein the cell surface marker is selected from the group consisting of CD30, EpCAM (CD326), CD9, Thy-1 (CD90), SSEA-3, SSEA-4, TRA-1-60 or TRA-1-81. The transduction according to the present invention does not interfere with the pluripotency, i.e. present transduced hESCs and iPSCs remain undifferentiated, i.e. are able differentiate into all germ layer lineages.

The inventors found out that the desired targeted transduction of hESCs and iPSCs is substantially improved by using lentiviral vector particles pseudotyped with a truncated morbillivirus fusion (F) protein and a mutated and truncated morbillivirus hemagglutinin (H) protein further displaying a single chain antibody to a stem cell surface marker at its ectodomain. In this approach, the single chain antibody is responsible that only those stem cells are transduced which express the respective cell surface marker that is recognized by the antibody. In a preferred embodiment the single-chain variable fragment (scFv) which is fused to the ectodomain of the H protein is an anti-CD30, anti-EpCAM (CD326), anti-CD9, anti-Thy-1 (CD90), anti-SSEA-3, anti-SSEA-4, anti-TRA-1-60, or anti-TRA-1-81 coding sequence, most preferably anti-CD30 coding sequence. These coding sequences are particularly suitable since the expression of the cell surface markers CD30, EpCAM (CD326), CD9, Thy-1 (CD90), SSEA-3, SSEA-4, TRA-1-60, and TRA-1-81 is rapidly down-regulated upon differentiation of hESCs and iPSCs and the transduction does not interfere with the pluripotency, i.e. present transduced hESCs and iPSCs are able differentiate into all germ layer lineages.

Such transfected stem cells can be used for example as cellular vehicle for protein-supplement gene therapy and/or to direct the stem cells into particular lineages. Furthermore, insertion of lineage-specific reporter constructs would allow isolation of lineage-specific cells, and would allow drug discovery, target validation, and/or stem cell based studies of gene function and the like.

### Brief description of the Figures

Figure 1 shows the amino acid sequence of the scFV-antiCD30 (upper sequence lane) and changed amino acids in the corrected scFV-antiCD30ₒₚₜ (lower sequence lane). Critical amino acids in the scFv framework sequence are substituted by the underneath amino acid. Variable complementarity determining regions (CDRs) of the heavy chain variable domain (V_{H}, light gray) and the light chain variable domain (V_{L}, dark gray) and the linker between V_{H} and V_{L} (black) are indicated.

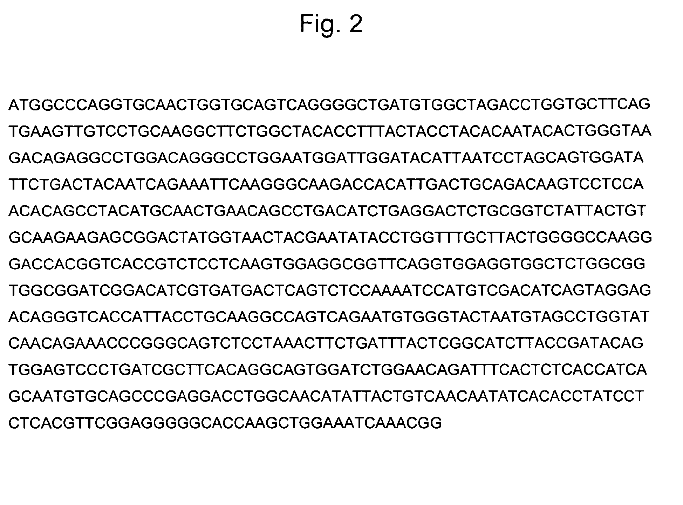

Figure 2 shows the corresponding DNA sequence to the corrected/optimized scFV-antiCD30 of Fig. 1

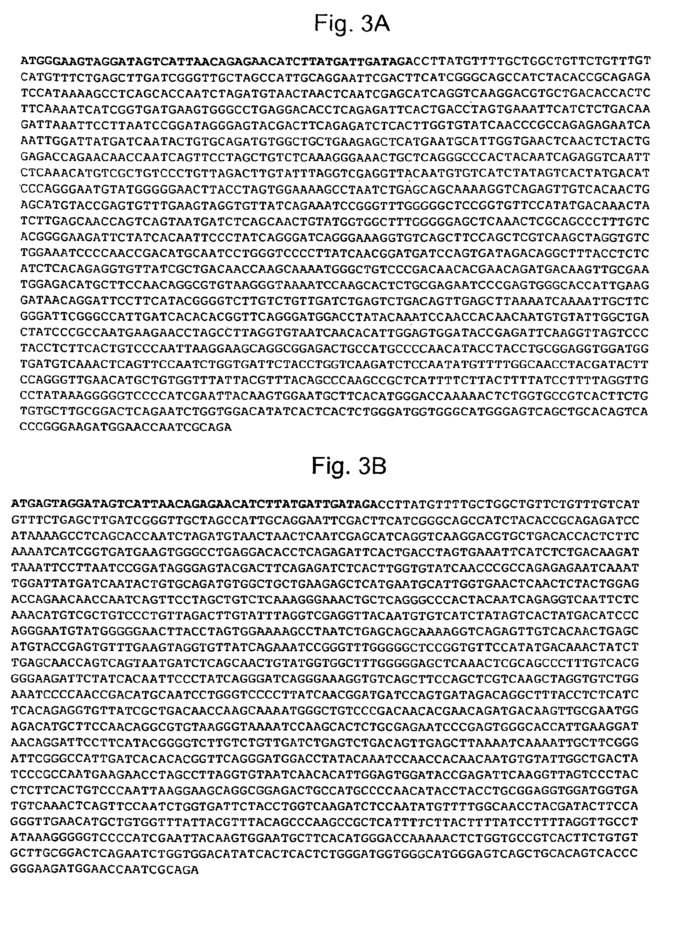

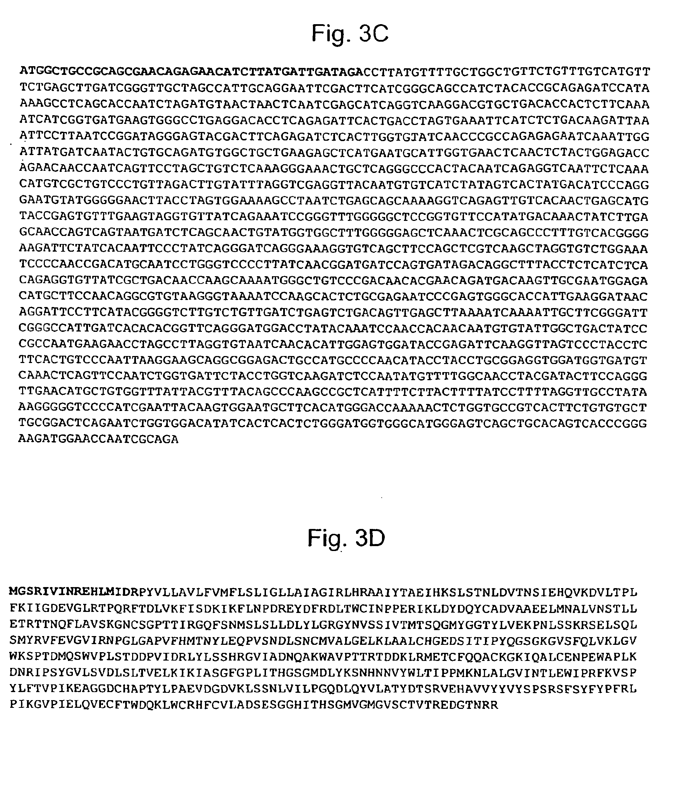

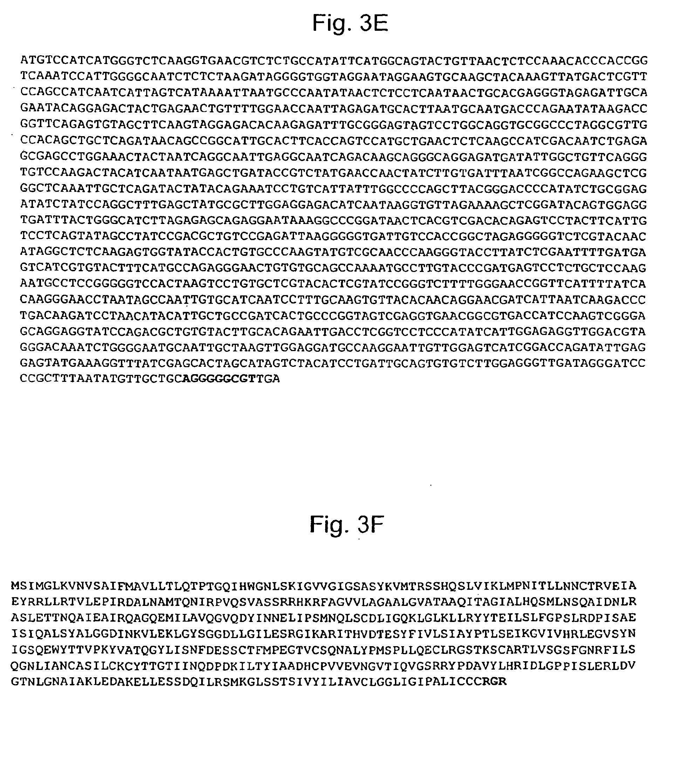

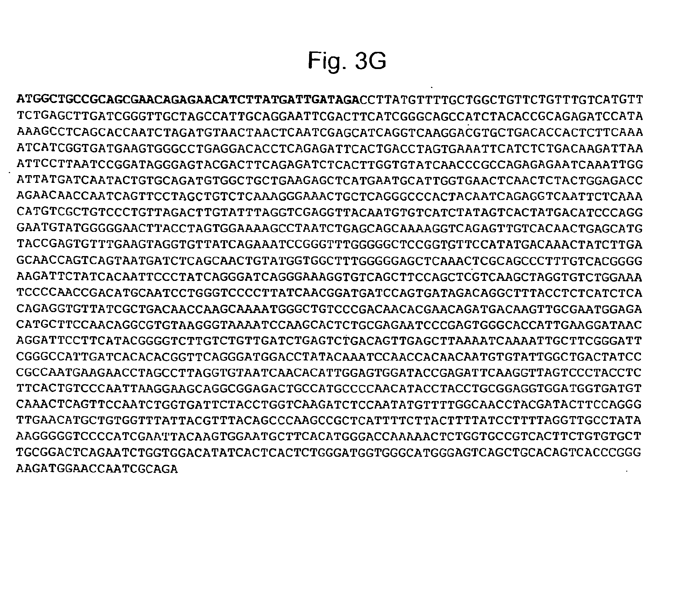

Figures 3A-3C exemplarily show the DNA sequence of three of the truncated H proteins derived from the wild-type (wt) DNA of the H protein of the Edmonton strain (H_{Edm}) of the measles virus (H_{Edm} wt DNA), namely, HcΔ18, HcΔ19 and HcΔ24+4A, respectively. Figure 3D exemplarily depicts the amino acid sequence of HcΔ18. Figures 3E and 3F exemplarily show the DNA and amino acid sequence of one of the truncated F proteins derived from the wild-type (wt) DNA of the F protein of the Edmonton strain of the measles virus (F_{Edm} wt DNA), namely FcΔ30. The cytoplasmic portions are shown in bold. Figure 3G depicts the DNA sequence of the truncated HcΔ24 protein, wherein additional four alanine residues were added to thus yield HcΔ24+4A. Again, the cytoplasmic portions are shown in bold.
Figures 4A and 4B provide an overview over exemplary generated F and H mutant proteins in comparison to their respective wt molecules, indicating the deleted (or added) amino acid residues in the cytoplasmic portion of the proteins.
Figures 5 depicts the amino acid sequence of the CD30ₒₚₜ.
Figure 6 shows exemplary microscopic pictures of iPSCs transduced with the pseudotyped lentiviral vector particles.

### Detailed description of the invention

The inventors developed novel pseudotyped lentiviral vector particles comprising a morbillivirus fusion (F) protein and a mutated hemagglutinin (H) protein of the measles virus (MeV) or the Edmonton strain of the measles virus (MeV_{Edm}), wherein the cytoplasmic portions of the F and the H protein are truncated, and wherein the amino acids necessary for receptor recognition in the H protein are mutated that it does not interact with CD46, SLAM and/or nectin-4 and further has a single chain antibody to a cell surface marker of hESCs and iPSCs at its ectodomain.

Furthermore, the inventors found out that the *in vitro* targeted transduction of undifferentiated pluripotent hESCs and iPSCs is substantially improved by using these lentiviral vector particles pseudotyped with a truncated morbillivirus fusion (F) protein and a mutated and truncated morbillivirus hemagglutinin (H) protein further displaying a single chain antibody to a stem cell surface marker at its ectodomain.

The term *"in vitro"* refers to studies in experimental biology that are conducted using components of an organism that have been isolated from their usual biological context in order to permit a more detailed or more convenient analysis than can be done with whole organisms. In contrast, the term *"in vivo"* refers to work that is conducted with living organisms in their normal, intact state, while "ex *vivo"* refers to studies on functional organs that have been removed from the intact organism.

The term "stem cell" refers to a cell that has the ability to self-renewal, i.e. to go through numerous cycles of cell division while maintaining the undifferentiated state, and has potency, i.e. the capacity to differentiate into specialized cell types, e.g. a nerve cell or a skin cell.

According to the present invention, "undifferentiated pluripotent stem cells" or rather "pluripotent stem cells" (PSCs) are able to generate all cell types of the body, but not those of the placenta, which is derived from the trophoblast and associated structures. In a preferred embodiment of the present invention, the PSCs are embryonic stem cells (ESCs), more preferably human embryonic stem cells (hESCs). The hESCs may be obtained from single blastomers of human embryos without embryo destruction. They may also be derived from established cell lines, which are commercially available.

An alternative pluripotent stem cell type is derived from *in vitro* cultured somatic cells that have been reversed into an embryonic-like pluripotent state, i.e. induced pluripotent stem cells (iPSCs). The iPSCs are also able to generate all cell types of the body, but not the umbilical cord, trophoblasts and associated structures. They functionally and phenotypically resemble embryonic stem cells. There are several publications and patents describing the reprogramming technique and suitable markers (e.g. WO 2010/033084 A1, WO 2009/144008 A1). The reprogramming according to the present invention is performed by introduction of stem cell-associated genes into non-pluripotent, i.e. differentiated cells, preferably fibroblasts, either by viral transduction or transfection. The stem cell associated genes are selected from the group consisting of Klf-4, Sox-2, Oct4, Nanog, LIN-28 and c-Myc, wherein 3-6 factors are introduced, preferably using a cocktail of 4 factors (including Oct4, Sox-2, Nanog and LIN-28).

Preferable hES stem cell lines of the present invention include H9 or HES-3.

Preferable iPS stem cell lines include hCBEC (obtained via lentiviral gene transfer), hFFBiPS SB4 and hFFBiPS SB5 (obtained via "Sleeping Beauty" transposition), wherein the iPS stem cell lines of the present invention can be also generated from diseased patients, such as from patients having adenosine-deaminase deficiency-related severe combined immunodeficiency (ADA-SCID), Duchenne (DMD) and Becker muscular dystrophy (BMD), Parkinson disease (PD) or juvenile-onset type 1 diabetes mellitus (JDM). For gene therapy, these iPS stem cells have to be transduced with an intact copy of the defective gene and can then, upon differentiation into the relevant cell type, be transferred to patients without causing an immune response, as already described by Zhao, T. et al. (Nature, 2011, 474: 212-215). Furthermore, these iPS stem cells can be used as a model system for therapeutic approaches of gene therapy. After specific transduction of these iPS cells, the expression of the therapeutic gene can be monitored before differentiation and effects of different expression rates can be detected during differentiation. In a preferable embodiment of the present invention, iPS cells, derived from ADA-SCID patients, are transduced with the CD30-LV vector, thus, transferring the functionally active adenosine-deaminase (ADA) gene. The cells are then differentiated towards the haematopoietic lineage, wherein CD30-positive cells are collected and analysed for expression of ADA. These cells can then be transplanted into immunodeficient mice where they engraft and differentiate into all lineages of the haematopoietic system

As used herein, the term "multipotent" refers to the ability to only differentiate into a limited number of types. For example, the bone marrow contains multipotent stem cells that give rise to all the cells of the blood but not to other types of cells. Multipotent stem cells are found in adult organisms, e.g. most organs of the body (e.g. brain, liver, heart) where they can replace dead or damaged cells.

"Lentivirus" (or "lentiviral") as used in the present invention is a genus of the *Orthoretroviridae,* a sub-family of the *Retroviridae,* wherein the viruses of the taxonomic family *Retroviridae* share, as a main feature, a positive stranded ssRNA genome (ss(+)RNA). The genetic information of these viruses is first reverse-transcribed from RNA into DNA by a reverse transcriptase provided by the virus and subsequently integrated into the host genome. Thus, the term "retro" refers to the activity of reverse transcriptase and the transfer of genetic information from RNA to DNA. As the viral genome is ss(+)RNA (mRNA) it can also directly be used by the cell as template for protein production,

According to the present invention, the lentivirus (LV) is selected from the group consisting of the human immunodeficiency virus (HIV), the bovine immunodeficiency virus (BIV), the feline immunodeficiency virus (FIV), the simian immunodeficiency virus (SIV) and equine infectious anemia virus (EIAV), more preferably HIV-1, HIV-2, SIV-_{mac}, SIV_{pbj}, SIV_{agm}, FIV and EIAV, and even more preferably HIV-1.

With regard to the detailed description of elements of the lentivirus, reference is made to the corresponding definitions in WO 2008/037458 A2, which are incorporated for reference herewith.

In one preferable embodiment of the present invention, three basic components on separate plasmids, for example on a protein expression plasmid (e.g. measles virus H or F protein expression plasmid), packaging plasmid (e.g. pCMVdR8.9), and/or transfer vector plasmid (e.g. pSEW), are provided for generating lentiviral vector particles: a psi-negative *gag*/*pol* gene, a psi-negative *env* gene and a psi-positive expression vector. In another preferred embodiment, the psi-positive lentiviral expression vector and/or the psi-negative lentiviral *gag*/*pol* gene are derived from a lentivirus selected from the group consisting of HIV-1, HIV-2, SIV_{mac}, SIV_{pbj}, SIV_{agm}, FIV and EIAV, and even more preferably HIV-1.

"Lentiviral vector particles", "pseudotyped lentiviral vector particles" or rather "vector particles" as used in the present invention are replication deficient lentiviral vectors that are useful for transducing a nucleic acid sequence into a genome of a host or target cell. Examples of pseudotyped lentiviral vector particles are described in detail in WO 2008/037458 and EP 11 151 018.6 to which reference is made. Such vector particles only contain an incomplete genome of the lentivirus from which they are derived. In detail, the vector particles comprise a minimum of the Gag, Pol and Env proteins and an RNA molecule (which can be an expression vector). The Gag, Pol, and Env proteins that are needed to assemble the vector particle are derived from the retrovirus and are provided *in trans* by means of a packaging cell line, preferably human embryonic kidney 293 cells that contain the SV40 Large T-antigen (HEK-293T or 293T), human sarcoma cell line HT-1080 (CCL-121), lymphoblast-like cell line Raji (CCL-86), glioblastoma-astrocytoma epithelial-like cell line U87-MG (HTB-14), or T-lymphoma cell line HuT78 (TIB-161), more preferably HEK-293T. The RNA molecule or expression vector is usually derived from the genome of the original retrovirus, which means that it comprises all the necessary elements for an effective packaging into the resulting lentiviral vector particles *(inter alia* the psi element and long terminal repeats, LTRs). However, in order for the lentiviral vector particle of the present invention to be replication deficient, the RNA molecule does not comprise the genetic information of at least one of the *gag, env,* or *pol* genes itself, wherein the genes are either removed or the expression of their gene products is prevented, preferably by frame shift mutation(s).

In another preferable embodiment, the RNA molecule is not integrated into the host cell genome, due to a defective integrase or missing integration signals on the RNA. With this RNA the encoded protein is translated for a limited time within the transduced cell, facilitating differentiation into a certain lineage (Sarkis et al., Curr. Gene Ther. 2008, 8(6), pp. 430-437). Alternatively, no specific RNA is packaged, and the vectors can be used for specific protein transfer (Voelkel et al., PNAS USA 2010, 107(17), pp. 7805-7810).

Thus, in a preferable embodiment, the RNA molecule of the lentiviral vector particle does not comprise the genetic information of all three of said genes, i.e. the *gag, env,* or *pol* genes. In a further embodiment, the RNA molecule does not comprise the genetic information of other nonessential genes, additionally. In case of HIV-1, these unessential genes are selected from the group consisting of *tat, vif, vpr, vpu* and *net.* Instead, a normally heterologous nucleic acid sequence to be integrated into the target/host genome ("transgene", i.e. a gene to be transduced) is present in the expression vector that is under control of a suitable promoter, and is thus expressed upon integration of the gene into the genome of the host or target cell. The term "suitable promoters" according to the present invention refers to promoters, which regulate the expression of housekeeping genes that are constitutively expressed in any tissue, for example the EF1A1 gene encoding the α-subunit of the eukaryotic elongation factor 1 or the gene encoding the phospho-glycerat kinase, and which show only a mild down regulation upon stem cell differentiation. Thus, the promoter of the present invention can be selected from the group consisting of the cytomegalovirus (CMV)-promoter, the spleen focus forming virus (SFFV)-promoter, the elongation factor 1 alpha (EF1α)-promoter (the 1.2 kb EF1α-promoter or the 0.2 kb EF1α-promoter), the chimeric EF1α/IF4-promoter, and the phospho-glycerate kinase (PGK)-promoter, wherein the 1.2 kb EF1 α-promoter and the SFFV-promoter are preferred.

In a further preferred embodiment, the lentiviral vector particle of the invention further comprises a psi-positive RNA expression vector comprising the transgene. Preferably, the psi-positive RNA expression vector comprises at least one selectable marker gene as the transgene.

In general, the terms "selectable marker" or "selectable marker gene", as used in the present invention, refers to a gene and its corresponding product that allow detection, selection and/or isolation of said product. If such a selectable marker is expressed by a cell, consequently such a cell or a population of such cells can be detected, selected and/or isolated. Selectable markers according to the present invention can be, *inter alia,* a molecule, preferably a peptide or protein, detectable by fluorescence, an enzyme catalyzing a reaction of which the resulting product is monitored, or a molecule that confers a resistance to, *inter alia,* an antibiotic. Detection, selection and/or isolation can be carried out, *inter alia,* by fluorescence activated cell sorting (FACS), a FACS cell sorter, use of fluorescence microscopy, use of immunofluorescence microscopy with primary and secondary antibodies, or by the use of antibiotics. Thus, in one preferred embodiment of the present invention, said selectable marker gene is selected from the group consisting of a gene coding for GFP (green fluorescent protein), a gene coding for eGFP (enhanced GFP), a gene coding for an apoptosis-inducing protein, a gene coding for a cytotoxic protein, TNF-α gene, p53 gene, an interfering RNA, an interferon gene, the herpes virus thymidine kinase gene, and a gene coding for an immune stimulatory or therapeutic protein.

Thus, in a preferable embodiment of the present invention, the lentiviral vector particles is based on a vector selected from the group consisting of HIV-1, HIV-2, SIV_{mac}, SIV_{pbj}, SIV_{agm}, FIV and EIAV, and even more preferably HIV-1. In a further embodiment, the RNA molecule of the lentiviral vector particle is based on the HIV-1 genome and comprises the LTRs, the psi element and the CMV promoter followed by the gene to be transduced, wherein the *gag, env, pol, tat, vif, vpr, vpu* and *nef* genes of HIV-1 are removed or expression of their gene products is prevented, and wherein the gene to be transduced is a selectable marker gene that is selected from the group consisting of a gene coding for GFP, a gene coding for eGFP, a gene coding for an apoptosis-inducing protein, a gene coding for a cytotoxic protein, TNF-α gene, p53 gene, an interfering RNA, an interferon gene, the herpes virus thymidine kinase gene, and a gene coding for an immune stimulatory or therapeutic protein.

For generating the lentiviral vector particles of the present invention, the three basic components, usually provided on separate plasmids, are required: a psi-negative *gag*/*pol* gene, a psi-negative env gene and a psi-positive expression vector. Co-transfection of these components into a packaging cell line, i.e. a suitable eukaryotic cell line, for example HEK-293T, leads to the expression of the Gag, Pol and Env proteins as well as generation of psi-positive RNA. Since the *gag*/*pol* and *env* genes of the present invention are psi-negative, the mRNA generated prior to expression of the corresponding proteins is not assembled into the lentiviral vector particles. Contrasting this, the psi-positive RNA transcribed from the expression vector of the present invention is included into the resulting lentiviral vector particle.

Consequently, the replication deficient lentiviral vector particles of the present invention are generated comprising the Gag, Pol and Env proteins provided *in trans* by the packaging cell line, preferably HEK-293T, as well as the psi-positive RNA transcript of the expression plasmid lacking the genetic information for autonomous replication. However, as the lentiviral vector particles comprise the Gag, Pol and Env proteins, they are able to efficiently infect their target/host cells, reverse-transcribe their RNA, and integrate said genetic information into the genome of the target/host cell.

For further details and conditions reference is made to WO 98/037458 A2, in particular the Example part thereof.

The term "pseudotyped", "pseudotyped vector" or "pseudotyped vector particle", "pseudotyped lentiviral vector particle" as used in the present invention, refers to a vector particle bearing envelope glycoproteins derived from other viruses having envelopes. The host range of the lentiviral vectors or lentiviral vector particles of the present invention can thus be expanded or altered depending on the type of cell surface receptor used by the glycoprotein, since such vector particles possess the tropism of the virus from which the glycoprotein(s) were derived.

Thus, in one preferable embodiment of the present invention, the *env* gene of the pseudotyped lentiviral vector particle, that is originally derived from the same retrovirus as the *gag* and *pol* genes and as the RNA molecule or expression vector, is exchanged for the envelope protein(s) of a different enveloped virus. In a preferred embodiment of present invention, said envelope protein(s) are the fusion (F) and the hemagglutinin (H) glycoproteins of a paramyxovirus or a morbillivirus, preferably the measles virus (MeV), or the Edmonton strain of the measles virus (MeV_{Edm}).

The MeV_{Edm} uses one of the following three receptors for cell entry, namely, the protein known to be the regulator of complement activation factor, CD46 (Gerlier, D. et al., Trends Microbiol., 1995, 3: 338-345), SLAM (signaling lymphocyte-activation molecule; also known as CD150) or Nectin-4, wherein the H protein, a type II transmembrane protein, binds to the MeV receptors CD46, SLAM or Nectin-4 , and the F protein, a type I transmembrane protein, carries a hydrophobic fusion peptide that mediates membrane fusion upon receptor binding of H.

Thus, one known function of the F protein is mediating the fusion of viral membranes with the cellular membranes of the host cell. Functions attributed to the H protein include recognizing the receptor on the target membrane and supporting F protein in its membrane fusion function. The mechanism of MeV-induced membrane fusion may involve receptor-induced conformational changes in the H and then F proteins, suggesting a dynamic interaction between these two proteins during the transfection process.

According to the present invention, the F and H proteins of the present invention are "truncated" at their "cytoplasmic portions". The term "cytoplasmic portion", "cytoplasmic tail" or "cytoplasmic region", as used in the present invention, refers to the portion of the respective protein that is adjacent to the transmembrane domain of the protein and, if the protein is inserted into the membrane under physiological conditions, extends into the cytoplasm.

The term "truncated" generally refers to a deletion of amino acid residues of the designated protein, wherein "truncated" also refers to the corresponding coding nucleic acids in a nucleic acid molecule that codes for a given "truncated" protein. Furthermore, it is to be understood that the nucleic acid molecules encoding for a specific truncated or modified protein of the present invention are likewise encompassed, and *vice versa.* For example, this means that if a given protein is referred to, e.g. a truncated F protein such as FcΔ30, the nucleic acid encoding said protein is likewise encompassed.

In the present invention, reference is specifically made to "truncated H" or "truncated F" proteins, which designate the morbillivirus, preferably MeV H and F proteins, respectively, whose cytoplasmic portion has been truncated, i.e. amino acid residues (or coding nucleic acids of the corresponding nucleic acid molecule encoding the protein) have been deleted. "HcΔX" and "FcΔX" designate such truncated H and F proteins, respectively, wherein "X" refers to 1-50 residues, that have been deleted of the cytoplasmic portion, respectively, more preferably 25-30 residues for FcΔX and 10-25 residues for HcΔX. Thus, in a further and even more preferred embodiment the "truncated F protein" is FcΔ24 or FcΔ30 and/or the "truncated H protein" is selected from the group consisting of HcΔ14, HcΔ15, HcΔ16, HcΔ17, HcΔ18, HcΔ19, HcΔ20, HcΔ21 +A, and HcΔ24+4A, more preferably HcΔ18, HcΔ19 or HcΔ24+4A.

In one preferable embodiment of the present invention, the truncated cytoplasmic portion of the F protein comprises at least one positively charged amino acid residue, wherein the truncated cytoplasmic portion of the H protein is truncated to allow efficient pseudotyping and has fusion support function. In a further preferred embodiment, the truncated cytoplasmic portion of the H protein comprises at least nine consecutive amino acid residues of the C-terminal cytoplasmic portion of the H protein plus an additional methionine at the N-terminus. In another and even more preferred embodiment, the truncated cytoplasmic portion of the F protein comprises at least three consecutive amino acid residues of the N-terminal cytoplasmic portion of the F protein and the truncated cytoplasmic portion of the H protein comprises at least 13 consecutive amino acid residues of the C-terminal cytoplasmic portion of the H protein plus an additional methionine at the N-terminus, wherein one to four of the N-terminal amino acid residues of said at least 13 consecutive amino acid residues of the C-terminal cytoplasmic portion of the H protein can be replaced by alanine residues. Furthermore, the cytoplasmic portion of the F protein of the present invention is located at the C-terminus of the protein.

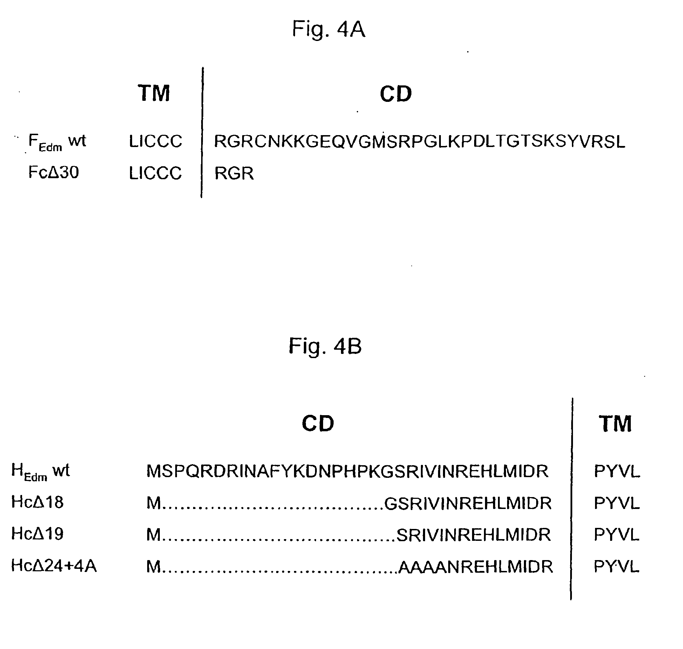

Thus, in a further and even more preferred embodiment, the one or two psi-negative expression vector(s) encode the truncated F protein FcΔ24 or FcΔ30 and/or the truncated H protein that is selected from the group consisting of HcΔ14, HcΔ15, HcΔ16, HcΔ17, HcΔ18, HcΔ19, HcΔ20, HcΔ21+A, and HcΔ24+4A, more preferably HcΔ18, HcΔ19 or HcΔ24+4A. In this regard reference is made to Figs. 3 and 4.

Normally, the lentiviral vector particles of the present invention as pseudotyped with the morbillivirus F and H proteins can be used to efficiently transduce cells (or cell lines) that carry at least one of the three MeV receptors, CD46, SLAM, and nectin-4. However, according to the present invention, the H protein is a truncated and mutated ("HcΔX", "Hmut" or rather "HmutΔX") protein that does not interact with CD46,SLAM, and/or nectin-4. In one embodiment of the present invention, the mutation for ablating/preventing interaction of the H protein with CD46 is introduced by the point mutations Y481A and R533A of the MeV H protein. In another embodiment, the Hmut protein also includes the mutations S548L and F549S, which lead to a more complete ablation of residual infectivity via CD46. Also, the mutation of the residues V451 and Y529 ablates productive interaction with CD46. Alternative mutations for ablating/preventing interaction of the H protein with CD46 relate to H proteins having Y481 replaced with any other amino acid, in particular with methionine or glutamine, or having mutations at one position selected from F431, V451, Y452, A527, P486, 1487, A428, L464, G546, S548, F549 wherein these amino acids are replaced with another amino acid and this mutation prevents or assists in preventing interaction of the H protein with CD46. Alternatively, replacement of all five consecutive residues 473 to 477 in H protein with alanine may prevent interaction of H protein with CD46. For preventing interaction of the H protein with SLAM one of the following residues may be replaced with any other amino acid, in particular, alanine: 1194, D530, Y553, T531, P554, F552, D505, D507. Furthermore, alternative mutations for ablating/preventing interaction of the MeV H protein with nectin-4 according to the present invention relate to the residues Y543 and P497, preferably Y543A or P497S and Y543A.

Thus, in a preferred embodiment, the H protein contains at least one mutation for ablating/preventing interaction of the H protein with at least one of the CD46 receptors CD46, SLAM and/or nectin-4, preferably the H protein contains two or three muttations.

In one embodiment, the mutation for ablating/preventing interaction of the H protein with CD46 is a point mutation with another amino acid of at least one of the residues selected from the group consisting of V451, Y529, Y481, F431, V451, Y452, A527, P486, 1487, A428, L464, R533, G546, S548, and F549, in particular with alanine, leucine, serin, methionine, glutamine, for example Y481A, R533A, S548L, and/or F549S; or Y481 replaced with methionine or glutamine. In an alternative embodiment, all five consecutive residues 473 to 477 are replaced with alanine in the H protein for ablating/preventing interaction of the H protein with CD46.

In another preferred embodiment, the mutation for ablating/preventing interaction of the H protein with SLAM is a point mutation with another amino acid, preferably alanine, at at least one of the residues selected from the group consisting of l194, D530, Y553, T531, P554, F552, D505, and D507.

In a further embodiment, the mutations for ablating/preventing interaction of the MeV H protein with nectin-4 relate to the residues Y543 and P497, preferably Y543A or P497S and Y543A.

Further details concerning the truncated F and H proteins and mutations can be derived from WO 98/037458 A2. Moreover, the person skilled in the art will readily be able to introduce mutations as, for example, additions and deletions, into a given nucleic acid or amino acid sequence. Such known methodologies are, for example, disclosed in Sambrook, J. et al., Molecular Cloning, A Laboratory Manual, 2nd ed., Cold Spring Harbor Laboratory (1989).

Furthermore, it has been found that the ratio between the amount of plasmid encoding the truncated F and H protein, respectively, used for the production of the pseudotyped lentiviral vector particles has a significant effect of the titers of the resulting vector particles. This means that an increased amount of plasmid coding for the truncated F protein compared to the amount of plasmid coding for the truncated H protein leads to both an increased titer and an increased transduction efficiency of the resulting pseudotyped lentiviral vector particle. In a preferred embodiment of the present invention, the amount of the truncated F protein is 10-100%, 100-250%, 250-500%, 500-750%, 750-1000%, or more than 1000% higher and, preferably, 300% higher than the amount of truncated H protein.

The terms "titers" and "transduction efficiency" as used in the present invention refer to the ability of a vector particle to enter a cell and integrate the genetic information, in particular RNA information, into the genome of the cell. Thus, the terms "titer" and "transduction efficiency" can be used synonymously. The titer/transduction efficiency can, for example, be determined by the incorporation of a gene coding for a selectable marker, for example, under the control of a CMV promoter, into the expression vector of the vector particle. Upon transduction of such selectable marker by means of the vector particle, the transduced cells will then express the selectable marker. If the selectable marker is, for example, the GFP protein, the number of cells infected by a given number of vector particles can readily be determined, e.g. by FACS analysis, and directly corresponds to the titer or transduction efficiency of the vector particles that have been used for transduction. The titer or transduction efficiency of a given vector particle thus refers to the number of cells transduced relative to the number of vector particles used. Thus, a vector particle suspension having an "increased titer" or an "increased transduction efficiency" is able to transduce a higher number of cells at a given vector particle concentration than a different vector particle suspension having the same vector particle concentration. In a preferable embodiment of the present invention, the titer of the virus stock is between 1 x 10⁶ and 1 x 10⁸ i.u./ml for the transduction of 1 x 10⁴ to 1 x 10⁶ iPS cells.

According to the present invention, the mutated and truncated H protein further displays a single-chain antibody at its ectodomain (i.e. at the C-terminus), i.e. H-scFv wherein the single-chain antibody is directed against or binding to a "cell surface marker" or rather "cell marker" that refers to a molecule present on the surface of a stem cell. It represents a fusion construct or in other words a chimeric protein. Such molecules can generally be, *inter alia,* peptides or proteins that may comprise sugar chains or lipids, antigens, clusters of differentiation (CDs), antigens, antibodies or receptors. Since not all populations of cells express the same cell markers, a cell marker can thus be used to identify, select or isolate a given population of cells expressing a specific cell marker. Thus, the terms "cell entry targeted" or "targeted" lentiviral vector particles as used in the present invention refer to pseudotyped lentiviral vector particles, wherein the H protein is mutated and further has a single-chain antibody to a cell surface marker at its ectodomain, i.e. by fusing the single-chain variable fragment (scFv) anti-cell surface marker coding sequence to the coding sequence of the H protein. Therefore, the host range of a cell entry targeted lentiviral vector particles of the present invention is not expanded or altered depending on the tropism of the virus the H protein is derived from, but, depending on the specificity of the single chain antibody fused to the H protein.

According to the present invention, the cell surface markers are selected from the group consisting of CD30, EpCAM (CD326), CD9, Thy-1 (CD90), SSEA-3, SSEA-4, TRA-1-60, and TRA-1-81, which are down regulated by differentiating stem cells. In a preferred embodiment, the mutated and truncated H protein further displays a single-chain antibody at its ectodomain that is directed against or binds to the cell surface marker CD30.

CD30 is a member of the tumour necrosis factor receptor family, originally identified as a surface marker for malignant cells in Hodgkin's disease (Schwab, U. et al., Nature, 1982, 299: 65-67). The biological function of CD30 in stem cells is still unknown, although its involvement in protecting stem cells against apoptosis was reported by Herszfeld, D. et al (Nat. Biotechnol., 2006, 24: 351-357). Furthermore, Grandela, C. & E. Wolvetang (Stem Cell Rev., 2007, 3: 183-191) observed that CD30-positive stem cells show a reduced expression of p53 (mediator or apoptosis) and an increased expression of *SURVIVIN* (inhibitor of apoptosis).

With regard to its expression, it has been reported that CD30 is strongly expressed in embryonal carcinomas (Pera, M. F. et al., Lab. Invest., 1997, 76: 497-504) but absent from most adult and embryonic tissue. Despite some contradicting results with respect to the CD30 expression in stem cells (Herszfeld, D. et al., Nat. Biotechnol., 2006, 24: 351-357; Grandela, C. & E. Wolvetang, Stem Cell Rev., 2007, 3: 183-191; Mateizel, I. et al., Human Reproduction, 2009, 24: 2477-2489) it is undisputed that CD30 expression is down-regulated during spontaneous differentiation of stem cells (Mateizel, I. et al., Human Reproduction, 2009, 24: 2477-2489; Lagarkova, M. A. et al., Cell Cycle, 2008, 7: 3610-3612). Thus, CD30 according to the present invention represents a marker of undifferentiated pluripotent hESCs and iPSCs.

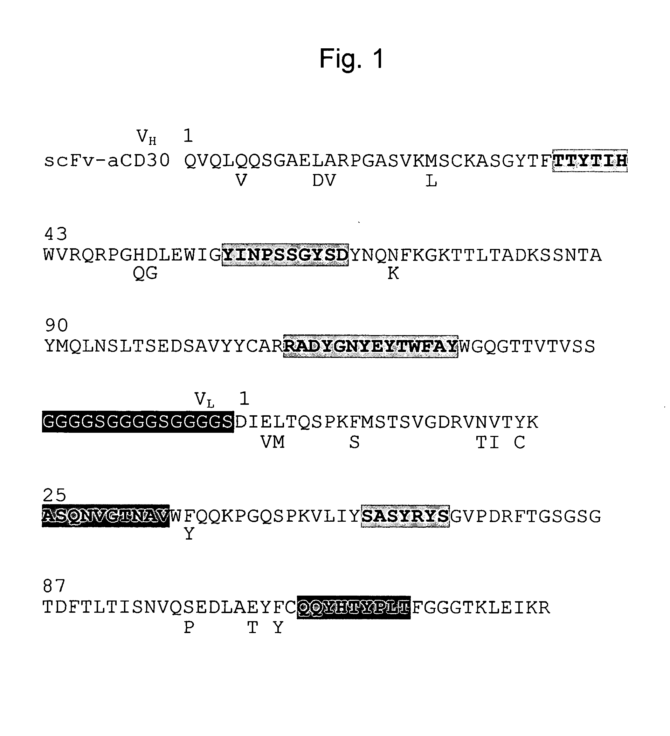

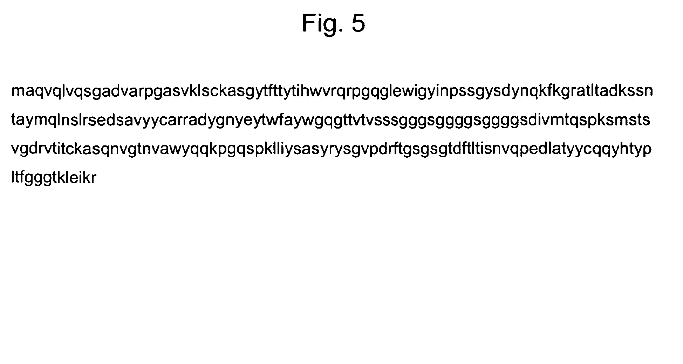

It was found by the present inventors that the known scFvCD30 should be preferably mutated to improve surface expression and the titre of the produced targeted lentiviral vector particle, preferably by point mutations at the amino acid position 5, 11, 12, 20, 50, 51, and/or 70 in the V_{H} chain, more preferably Q5V, L11D, A12V, M20L, H50Q, D51 G, and/or N70K; and at the amino acid positions 3, 4, 10, 20, 21, 23, 36, 98, and/or 104, more preferably E3V, L4M, F10S, N20T, V211, Y23C, F36Y, S98P, E103T, and/or F105Y in the V_{L} chain. The amino acid sequence of the scFv-antiCD30 and the corrected/optimized amino acid sequence are shown in Fig. 1 and 5.

The Epithelial Cell Adhesion Molecule (EpCAM) is a homophilic cell adhesion protein, mainly expressed on strongly replicating epithelia, like mucosa or basal skin layer. During embryogenesis, it is expressed on precursors of e.g. hepatocytes (De Boer, C. J. et al., J. Pathol., 1999, 188: 201-206) but not on adult cells. Its de *novo* expression on all carcinoma (Roovers, R. C., et al., Br. J. Cancer, 1998, 78: 1407-1416) is associated with dedifferentiation and enhanced proliferation of the tumour cells. Furthermore, the expression of EpCAM is associated with the maintenance of the undifferentiated phenotype of hES cells (Lu, T.-Y., et al., J. Biol. Chem., 2010 285: 8719-8732). Thus, EpCAM according to the present invention represents a marker of undifferentiated pluripotent hESCs and iPSCs.

The scFv recognising EpCAM is described by (Willuda, J. et al., Cancer Res., 1999, 59: 5758-5767). The H-scFv fusion protein cloning according to the present invention can be performed following to the procedure already described by Anliker, B. et al. (Nat. Methods, 2010, 7: 929-935), wherein the specific transduction of EpCAM-positive cells proves target specificity and appropriate titers.

CD9 (TG30) is a tetraspanin and described to be involved in cell adhesion (Masellis-Smith, A. & A. R. Shaw, J. Immunol., 1994, 152: 2768-2777), motility and metastasis (Ikeyama, S. et al., J. Exp. Med., 1993, 177: 1231-1237). Furthermore, it has a role in egg/sperm fusion (Higginbottom, A. et al., Biochem. Biophys. Res. Commun., 2003, 311: 208-214) and is listed by "The International Stem Cell Initiative" (Nat. Biotechnol., 2007, 25: 803-816) as marker for hES cells. Thus, CD9 is a cell surface protein known to function during cell development, growth and motility and is down regulated by differentiating hPSCs, whereby CD9 according to the present invention represents a marker of undifferentiated pluripotent hESCs and iPSCs.

Thy-1 (CD90) is a singlepass transmembrane protein with an Ig-like V-type (immunoglobulin-like) domain and is also described to be expressed on hematopoietic precursor cells (Craig, W. et al., J. Exp. Med., 1993, 177: 1331-1342). Thus, CD90 can be considered as a surrogate marker for various kinds of stem cells (e.g. hematopoietic stem cells or hES cells, including undifferentiated pluripotent hESCs and iPSCs

Stage-specific embryonic antigens 3 and 4 (SSEA-3 and SSEA-4) are two different epitopes on the same glycolipid exclusively present on hES or iPS cells (Thomson, J. A. et al., Science, 1998, 282: 1145-1147) or embryonal carcinoma cells, which are down regulated upon differentiation (Draper, J.S. et al., J. Anat., 2002, 200(Pt 3): 249-258). Thus, SSEA-3 and SSEA-4 according to the present invention represent marker of undifferentiated pluripotent hESCs and iPSCs.

In summary, the pseudotyped lentiviral vector particles of the present invention, with a truncated F protein (for example FcΔ30) and a mutated H protein (e.g. "HmutΔ18) additionally displaying a single-chain antibody to a cell surface marker at its ectodomain, for example scFvCD30, scFvEpCAM (scFvCD326), scFvCD9, scFvThy-1 (scFvCD90), scFvSSEA-3, scFvSSEA-4, TRA-1-60, or TRA-1-81, no longer enter cells via CD46,SLAM, and/or nectin-4, but are rather targeted to and enter only those cells displaying the respective corresponding markers at their surface. This means that the titers are significantly reduced on cells not expressing these markers. Therefore, cell entry and transduction using the pseudotyped lentiviral vector particles of the present invention represent efficient and effective means for highly selective gene transfer into specific cells, i.e. for the targeted transduction of specific cells. According to the present invention such cells are stem cells, more preferably undifferentiated pluripotent stem cells, wherein the stem cells are selected from the group consisting of hESCs and iPSC.

The term "differentiation" or "differentiation pathway" as used in the present invention refers to the pathway followed by a stem cell as it heads towards a lineage-specific or mature cell type and so begins with an undifferentiated stem cell and ends with a lineage-specific or mature, differentiated cell. A stem cell following such a pathway can go through many stages, wherein "undifferentiated pluripotent stem cells" or rather "pluripotent stem cells" are thought to have the potential to differentiate in almost any cell type, while "undifferentiated multipotent stem cells" or rather "multipotent stem cells" are believed to have the potential to differentiate into many cell types only. Since CD30, EpCAM (CD326), CD9, Thy-1 (CD90), SSEA-3, SSEA-4, TRA-1-60, and TRA-1-81 expression is undetectable in the areas of differentiation, CD30, EpCAM (CD326), CD9, Thy-1 (CD90), SSEA-3, SSEA-4, TRA-1-60, and TRA-1-81 expression reflects the undifferentiated state of the stem cells. Thus, the pseudotyped lentiviral vector particles of the present invention specifically transduce (i.e. targeted transduction) undifferentiated pluripotent CD30-, EpCAM (CD326)-, CD9-, Thy-1 (CD90)-, SSEA-3-, or SSEA-4-, TRA-1-60-, and TRA-1-81-positive stem cells, more preferably human embryonic stem cells (hESCs) and induced pluripotent stem cells (iPSCs), wherein present targeted transduction of these cells does not interfere with their pluripotency. Thus, depending on the transgene of the present invention, transgene silencing may be prevented which occurs over time or upon stem cell differentiation.

To summarize, the present invention is based on the unexpected and surprising finding that the incorporation of morbillivirus, preferably MeV, F and H proteins having truncated cytoplasmic tails into lentiviral vector particles, and the complex interaction of these two proteins during cellular fusion, allows for a superior and more effective transduction of cells, as the measles virus is characterized by direct and high efficient membrane fusion. Moreover, these pseudotyped vector particles allow the targeted gene transfer or rather targeted transduction of CD30-, EpCAM (CD326)-, CD9-, Thy-1 (CD90)-, SSEA-3-, SSEA-4-, TRA-1-60, and TRA-1-81-positive stem cells, more preferably human embryonic stem cells (hESCs) and induced pluripotent stem cells (iPSCs), as CD30, EpCAM (CD326), CD9, Thy-1 (CD90), SSEA-3, SSEA-4, TRA-1-60, and TRA-1-81 expression is rapidly down-regulated upon differentiation of hESCs and iPSCs. Thus, the titres and the transduction efficiency of the pseudotyped lentiviral vector particles of the present invention are significantly increased and only multipotent stem cells are transduced, so that the transgenes are not silenced upon stem cell differentiation. Targeting the molecule CD30, EpCAM (CD326), CD9, Thy-1 (CD90), SSEA-3, SSEA-4, TRA-1-60, or TRA-1-81 with CD30-, EpCAM (CD326)-, CD9-, Thy-1 (CD90)-, SSEA-3-, SSEA-4-, TRA-1-60, or TRA-1-81-specific lentiviral vectors is very efficient and strictly specific. As shown in the example part, it was possible with this approach that only iPSC colonies expressing Oct4 (a well known stem cell marker) express the marker gene (e.g. GFP) after transduction with CD30-, EpCAM (CD326)-, CD9-, Thy-1 (CD90)-, SSEA-3-, SSEA-4-, TRA-1-60, or TRA-1-81-lentoviral vector particles. Other stem cell makers can be included in the embodiments of the present invention, e.g. Sox-2, Nanog as well as expression and activity of alkaline Phosphatase (an enzyme, which is only active in pluripotent cell types). This is an huge advantage since the prior art transduction technologies cannot discriminate stem cells neither from feeder cells nor from differentiated cells, as they normally occur during the cultivation of iPSCs.

The present invention is particularly suitable for basic research, e.g. functional genomics, as well as therapeutic applications of iPSCs. The invented method is faster, easier to handle and gentler for the cells than state of the art protocols. Moreover, fewer cells will be required. With regard to iPSCs which are a non-homogenous mixed cell population composed of pluripotent cells, differentiated and undifferentiated cells it is an advantage that with the present invention it is possible to distinguish between cells expressing certain markers (e.g. CD30, EpCAM (CD326), CD9, Thy-1 (CD90), SSEA-3, SSEA-4, TRA-1-60 or TRA-1-81) and to selectively and specifically transduce these cells, wherein present targeted transduction does not interfere with the pluripotency, i.e. present transduced hESCs and iPSCs are able to differentiate into cell types of all three germ layers which can be tested using different germ layer marker genes, for example β-tubulin or cytokeratin-18 for ectoderm; α-fetoprotein (AFP), sex determining region Y-box 17 (SOX-17) or hepatocyte nulear factor-4 (HNF4) for endoderm; desmin or CD31 for mesoderm.

The invention is further described in the following Examples which, however, are not construed to limit the invention.

### Examples

### Example 1: Production and Isolation of pseudotyped lentiviral vector particles displaying scFV-CD30

As described in WO/2008/037458, the scFv anti-CD30 coding sequence was genetically fused to the modified coding sequence of measles virus hemagglutinin (H) protein. The scFv framework was modified as described in the previous patent application EP 11151018.6. Clone pHL3-CD30mut2#2 mediated a high titer and was used for further experiments. The transfection method for the production of vector particles is described in Anliker, B. et al. (Nat. Methods, 2010, 7: 929-935). Briefly, HEK-293T cells were transfected using poly-ethylene-imine (PEI). For this purpose, 1.5 × 10⁷ cells were seeded 24 h before transfection into a T175 flask. Before transfection, the medium was replaced with 15 ml of Dulbecco's modified Eagle medium (DMEM) supplemented with 2 mM glutamine. In total, 2.6 µg of measles virus H protein expression plasmid, 8 µg of measles virus fusion (F) protein expression plasmid, 28.7 µg of packaging plasmid pCMVdR8.9 and 30.2 µg of transfer vector plasmid pSEW were mixed with 310 µl of 5% (wt/vol) glucose. In parallel, 70 µl of 18 mM PEI and 310 µl of 5% glucose were mixed. Both mixtures were incubated for 10 min at room temperature (25°C), combined vortexed and incubated for additional 10 min at room temperature. After adding 1 ml DMEM supplemented with 2 mM glutamine, the mixture was added to the cells. After 4 h, 2 ml of FCS were added. The cell supernatant containing the pseudotyped particles was filtered 48 h later (0.45-µm filter) and concentrated by ultracentrifugation over a 20% (wt/vol) sucrose cushion (100,000 g for 3 h at 4°C). The supernatant was discarded and the pellet was resuspended in 150 µl reduced serum medium (Opti-MEM) or phosphate buffered saline (PBS). Vector particle stocks were titrated on HuT78 cell line. eGFP expressing cells (eGFP⁺ cells) were determined by FACS analysis and titers were calculated..

Similar experiments were carried out using clone pHL3-CD30ₒₚₜ.which showed similar good titers as the above mentioned CD30mut#2.

### Example 2: Cultivation and Transduction of iPSCs

iPS cells are maintained on an inactivated feeder layer of murine embryonic fibroblasts in the presence of 20% Knock-Out (KO) Serum replacement, 1 mM non-essential amino acids, 1 mM L-glutamine, 0.1 mM β-mercapto-ethanol, 50 U penicillin/50 µg streptomycin (Pen/Strep) and 8 ng/ml basic fibroblast growth factor (bFGF or FGF2) in KO-DMEM. For subcultivation, medium was removed and collagenase (2 mg/ml Collagenase Type IV in KO-DMEM) was added for 5 min to dissolve the stem cell colonies from the feeder layer. Subsequently, 1 ml medium was added to wash the colonies from the feeder layer and the colonies were concentrated by centrifugation (3 min, 800 rpm, 4°C). The cell pellet was resuspended in an appropriate amount of fresh medium and allocated to fresh feeder plates.

For the transduction of iPS cells, an appropriate amount of colonies was seeded two days before transduction. To 1 ml cell culture medium, 10 µl of vector stock (titer: 1 x 10⁷ i.u./ml) was added resulting in a multiplicity of infection (MOI) of 2 when 5 x 10⁴ iPS cells are present in the well. Alternatively, the vector stock was added to the cells before allocation to the fresh feeder plates.

After transduction, the cells were cultivated for another 4 days until GFP expression derived from the transferred reporter gene became detectable. A reasonable number of transduced cells were already detectable after adding the CD30-LV directly to the cultured cells. Cells were fixed with 4% formaldehyde in PBS at room temperature for 15 min for immunofluorescence analysis. The marker Oct4 was visualised in parallel to the GFP expression by an Oct3/4 antibody from Santa Cruz (incubation at room temperature for one hour) and a secondary goat-anti-mouse-antibody (Alexa-647; 1:1000) after an incubation at room temperature for 30 min.

### Example 3: Cell entry targeted lentiviral vector particles directed to CD30-expressing cells

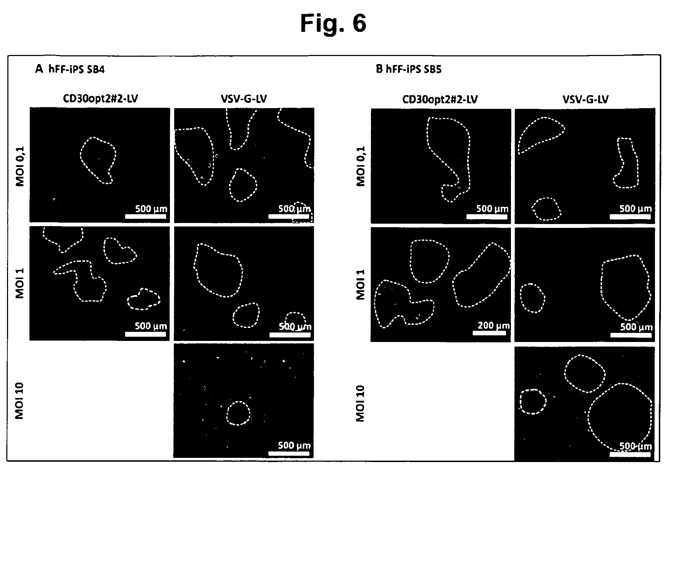

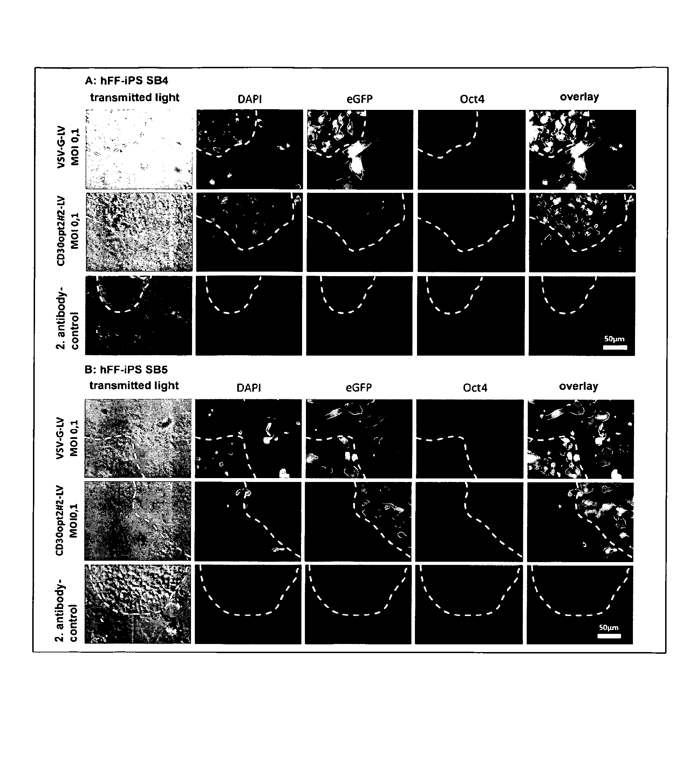

1.6 x 10⁵ of the iPS cell lines hFFBiPS SB4 and hFFBiPS SB5 were seeded on a feeder layer of mouse embryo fibroblasts in a 24-well-plate. Transduction followed 24 h later with vesicular stomatitis virus G protein (VSV-G) pseudotyped lentiviral vectors (VSV-G-LV) and CD30ₒₚₜ-LV (MOI 0.1; 1 and 10 [only VSV-G-LV]) in 500 µl stem cell medium. After 6 h, medium was changed and cells were cultivated for 120 h. The eGFP-expression of transduced cells was analysed by fluorescence microscopy (Figure 6A). For immunofluorescence pictures showing Oct4, CD30 or SSEA-4, cells were seeded on coverslips and transduced at an MOI of 0.1. Then the cells were washed with PBS once, fixed for 15 min with 4% formaldehyde in PBS and permeabilised with 1% Triton X-100 for 10 min at room temperature (RT). Unspecific binding sites were blocked with 5% bovine serum albumin (BSA)/0.1 % Triton X-100 in PBS for 30min at RT. The primary antibodies were used 1:500 in PBS with 5% BSA and incubated with the cells for 1 h at RT. Cells were washed three times with PBS for 5 min then incubated with the according secondary antibodies (diluted 1:1000 in PBS) for 30 min at RT in the dark. After washing with PBS (three-times, 5 min) nuclei were stained with 4'6-diamidino-2-phenylindole (DAPI) (1 µg/ml in PBS) for 1 min and washed again three-times for 10 min. Coverslips were mounted on slides with Aquapolymount (Polysciences Europe GmbH, Eppelheim, Germany) and stored at 4°C until analysis.

### Example 4: Different promotors suitable for transgene expression after differentiation

Transduction of iPS cells with lentiviral targeting vectors should not interfere with the pluripotency of the cells. Therefore, transduced cells must be demonstrated to differentiate into all germ layer lineages. This can be demonstrated by embryoid body formation in cell culture, or teratoma formation after injection into immundeficient mice. Besides the SFFV-promoter that is silenced during differentiation, four different promoters have been tested: the 1.2 kb EF1α-promoter, the chimeric 0.4 kb EF1α/IF4 promoter, the short 0.2 kb EF1α-promoter and the PGK-promoter. To test the expression of a marker gene under control of these different promoters, 1 x 10⁵ cell lines derived from different human tissues (293T, HT1080, Raji, U87-MG and HuT78) have been transduced with VSV-G-LV at an MOI of 0.1 and analyzed GFP-expression after 96 h. In all cell lines, the 1.2 kb EF1α-promoter showed a similar strong activity as the SFFV-promoter and was used for iPS cell transduction.

### Example 5: Differentiation of iPS to embryoid bodys and lineages of all three germlayers after marker gene transfer

According to a protocol of Cerdan, C. et al. (Curr. Protoc. Stem Cell Biol. 2007, 1:1 D.2.1-1 D.2.16), iPS cells were grown in six-wells on the feeder-layer in 3 ml embryonic stem cell (ESC) medium (40 ml KnockOut-DMEM, 10 ml KnockOut Serum Replacement, 0.5 ml MEM non-essential amino acids, 0.25 ml L-glutamine, 0.25 ml Pen/Strep, 0.1 µl 2-mercaptoethanol, 4 ng/µl FGF2) until they form colonies and express Oct4 uniformly. When the iPS cells were ready for subcultivation, differentiation was started. To induce the formation of embryoid bodies (EBs), iPS cells in one six-well plate were detached from the feeder using 1 mg/ml collagenase (Type IV). For this purpose, the medium was aspirated, 1 ml collagenase was added to each well and the cells were incubated for 5 min at 37°C. 2 ml ESC medium was added to each well and the colonies were carefully detached from the feeder using a 1 ml pipette. The colonies were transferred to a 15 ml tube and centrifuged for 3 min at 800 rpm. The supernatant was aspirated and the iPS cells were resuspended in EB medium (80 ml KnockOut DMEM, 20 ml FCS, 0.5 ml L-glutamine, 0.67 µl 2-mercaptoethanol, 1 ml ascorbic acid (5 mg/ml), 0.67 ml holo-transferrin (30 mg/ml). 3 ml of cell suspension were transferred to ultra-low attachment plates and incubated at 37°C. Medium was exchanged every 2 days. The general formation of EBs was stopped after 10 days of differentiation and expression of GFP was monitored. To monitor the expression of marker genes for all three germ layers RNA was extracted at different time points during differentiation and reverse transcriptase (RT)-PCR performed with primer pairs specific for different germ layer marker genes (ectoderm: β-tubulin, cytokeratin 18; endoderm: AFP, SOX-17, HNF4; mesoderm: desmin, CD31).

To induce differentiation of iPS cells to spinal motoneurons a protocol published by Hu, B. Y. & S. C. Zhang (Nat. Protoc., 2009, 4:1295-1304) is applied. For the differentiation the following steps have been used: Induction of primitive neuroectoderm (NE) cells (days 0-10), specification of the basic helix-loop-helix transcription factor Olig2-expressing motoneuron progenitors (days 10-28), generation of spinal motor neurons by differentiating on the substrate (days 28-35) and maturation of spinal motor neurons on the substrate (days 42-56). For this purpose, iPS cells were detached from the mouse embryonic fibroblast (MEF) feeder layer using collagenase type IV, collected in a 15 ml tube and centrifuged for 1 min at 50 g and room temperature. The colonies were then transferred to T75 flasks and cultured at 37°C in ESC medium without FGF2. Medium was exchanged every second day. On day 7 medium was exchanged for neural differentiation medium (484 ml DMEM medium with nutrient mixture F-12 (DMEM/F12), 5 ml of serum free supplement based on Bottenstein's N-1 formulation (N-2 supplement), 5 ml MEM non-essential amino acids, 5 ml Pen/Strep, 1 ml heparin (1 mg/ml)). 20-25 clusters (EB's) were seeded on a laminin-coated 12-well plate in 300 µl neural differentiation medium containing retinoic acid (0.1 µM) and sonic hedgehoc homolog (SHH) protein (100 ng/ml). The following day 2 ml of neural differentiation medium was added and the clusters cultured at 37°C. On day 10 medium was changed to neural differentiation medium containing retinoic acid (0.1 µM). On day 15 neural tube-like rosettes were carefully detached from the plate, transferred to a new cell culture T75 flask containing neural differentiation medium and retinoic acid. Medium was exchanged every second day. On day 20 the clusters were transferred to a 15 ml tube and centrifuged for 2 min at 50 g and room temperature. After removal of the medium the clusters were incubated with 1 ml Accutase until the suspension looks cloudy. After addition of 9 ml neural differentiation medium the cells were pelleted and the clusters were downsized by careful pipetting. The smaller clusters were transferred to a new cell culture T75 flask. Medium was exchanged every second day. On day 28 generation of spinal motor neurons was induced by seeding 3-5 spheres on poly-L-ornithin/laminin coated plates in the presence of brain-derived neutrophic factor (BDNF), glial cell line-derived neutrophic factor (GDNF), insulin-like growth factor 1 (IGF-1) (10 ng/ml), cAMP (1µM), ascorbic acid (200 ng/ml), retinoic acid (50 nM) and SHH (50 ng/ml). Medium was exchanged every second day until day 56. On day 35 some cells were stained for the presence of HB9. On day 42 the expression of choline acetyltransferase (ChAT) was evaluated. On day 56 immunostaining for synapsin 2 was performed.

To generate hepatocytes, a protocol published by Moore, R. N. & P. V. Moghe (Stem Cell Res., 2009, 3: 51-62) was used. Briefly, the iPS cells were differentiated by cultivation on MEFs or Matrigel-coated plates in the presence of various growth factors over a 2-weeks period. For the differentiation, cells were grown in stem cell medium without FGF2 but supplemented with dexamethasone (10-7 M), oncostatin M (10 ng/ml), hepatocyte growth factor (20 ng/ml), activin A (50 ng/ml) and Wnt 3A (100 ng/ml) for two weeks with daily exchange of the medium. To determine the extent of differentiation the cells were stained for hepatospecific markers (AFP, albumin, cytokeratin 18 (CK18), alpha-1-antitrypsin (AAT), HNF-4, forkhead box A2 (FOXA2), inducible cytochrome P450 1A2 (CYP1A2)) or analysed by RT-PCR. Pure populations of hepatocytes were generated by FACS sorting. Functionality of the hepatocytes was determined by an albumin secretion analysis using an enzyme-linked immunosorbent assay (ELISA assay) as well as by an ethoxyresurofin-*O*-deethylase (EROD) assay to monitor CYP1A2 activity via the conversion of ethoxyresorufin to resorufin according to the method described in Moore, R. N. & P. V. Moghe (Stem Cell Res., 2009, 3: 51-62).

For the generation of cardiomyocytes, a protocol adapted from Laflamme, M. A. et al. (Nat. Biotechnol., 2007, 25: 1015-1024) was used. For the induction of cardiac differentiation pluripotent cells were plated on Matrigel-coated plates and cultivated in RPMI1640 plus B27 supplement (RPMI-B27) medium including 100 ng/ml human recombinant activin A for 24h, followed by 10 ng/ml human recombinant bone morphogenic protein 4 (BMP4) for 4 days. Then the medium was changed to RPMI-B27 without supplementary cytokines for 2-3 additional weeks with medium exchange every second day. To determine the differentiation status of the cells, RT-PCR analysis of several cardiac mesodermal marker genes (transcription factor GATA-4, homeobox protein-coding gene Nkx2.5, T-box transcription factor-coding genes Tbx5 and Tbx20; myosin light chain (MLC) kinases MIc2a and MIc2v) was performed. The expression of atrial natriuretic factor (ANF), myosin light chain 2 (MIc2), myosin heavy chain (MHC), troponin T, Titin, ANF and sarcomeric α-actinin were evaluated by immunofluorescence staining. Pure populations of cardiomyocytes were isolated by FACS sorting.

## Claims

1. A pseudotyped lentiviral vector particle, comprising a morbillivirus fusion (F) protein and a mutated hemagglutinin (H) protein of the measles virus (MeV) or the Edmonton strain of the measles virus (MeV_{Edm}),
wherein the cytoplasmic portions of the F and the H protein are truncated, wherein the amino acids necessary for receptor recognition in the H protein are mutated that it does not interact with CD46, SLAM and/or nectin-4 and further has a single chain antibody to a cell surface marker of hESCs and iPSCs at its ectodomain.

2. The pseudotyped lentiviral vector particle according to claim 1, wherein the single chain antibody is directed against CD30, EpCAM (CD326), CD9, Thy-1 (CD90), SSEA-3, SSEA-4, TRA-1-60, or TRA-1-81.

3. The pseudotyped lentiviral vector particle according to claim 1 or 2, wherein the mutation in the H protein is:
- at least one point mutation with another amino acid at the residues selected from the group consisting of V451, Y529, Y481, F431, V451, Y452, A527, P486, 1487, A428, L464, R533, G546, S548, and F549 with alanine, leucine, serin, methionine, or glutamine; and/or
- a point mutation of all five consecutive residues 473 to 477 with alanine;
and/or
- at least one point mutation with another amino acid at the residues selected from the group consisting of I194, D530, Y553, T531, P554, F552, D505, and D507; and/or
- at least one point mutation with another amino acid at the residues selected from the group consisting of Y543 and P497.

4. The pseudotyped lentiviral vector particle according to any of claims 1 to 3, wherein the scFv is directed against CD30 having the optimized amino acid sequence as shown in Fig. 5.

5. The pseudotyped lentiviral vector particle according to any of claims 1 to 4, wherein the truncated F protein is FcΔ24 or FcΔ30 and the mutated and truncated H protein is selected from the group consisting of HcΔ14, HcΔ15, HcΔ16, HcΔ17, HcΔ18, HcΔ19, HcΔ20, HcΔ21+A and HcΔ24+4A, preferably HcΔ18, HcΔ19, and HcΔ24+4A.

6. The pseudotyped lentiviral vector particle according to any of claims 1 to 5, wherein the pseudotyped lentivral vector particle is derived from a lentivirus selected from the group consisting of HIV-1, HIV-2, SIV_{mac}, SIV_{pbj}, SIV_{agm}, FIV and EIAV, preferably HIV-1.

7. The pseudotyped lentiviral vector particle according to claim 6, wherein the pseudotyped lentiviral vector particle does not comprise the genetic information of the gag, *env,* and/or *pol* genes and/or of other nonessential genes, which are selected from the group consisting of *tat, vif, vpr, vpu,* and *nef.*

8. The pseudotyped lentiviral vector particle according to claim 7, wherein said genes are provided in trans by means of a packaging cell line, which is selected from the group consisting human embryonic kidney 293 cells that contain the SV40 Large T-antigen (HEK-293T or 293T), human sarcoma cell line HT-1080 (CCL-121), lymphoblast-like cell line Raji (CCL-86), glioblastoma-astrocytoma epithelial-like cell line U87-MG (HTB-14), and T-lymphoma cell line HuT78 (TIB-161), more preferably HEK-293T.

9. The pseudotyped lentiviral vector particle according to any of claims 1 to 8, wherein the amount of the truncated F protein present in said vector particle is higher than the amount of mutated and truncated H protein present in said vector particle, preferably 10-100%, 100-250%, 250-500%, 500-750%, 750-1000%, or more than 1000% higher and, preferably, 700% higher than the amount of mutated and truncated H protein.

10. The pseudotyped lentiviral vector particle according to any of claims 1 to 9, further comprising a psi-positive RNA expression vector, wherein the psi-positive RNA expression vector comprises at least one selectable marker gene.

11. The pseudotyped lentiviral vector particle according to claim 10, wherein the selectable marker gene is selected from the group consisting of a gene coding for GFP, a gene coding for eGFP, a gene coding for an apoptosis-inducing protein, a gene coding for a cytotoxic protein, TNF-α gene, p53 gene, an interfering RNA, an interferon gene, the herpes virus thymidine kinase gene, a gene coding for an immune stimulatory protein and a gene coding for a therapeutic protein.

12. Use of the pseudotyped lentiviral vector particle according to any of claims 1 to 11, in the *in vitro* targeted transduction of undifferentiated pluripotent human embryonic stem cells (hESCs) and induced pluripotent stem cells (iPSCs).

13. Use according to claim 12, wherein the hESCs are selected from the group consisting of H9 and HES-3 and the iPSCs are selected from the group consisting of hFFBiPS SB4 and HFFBiPS SB5 or are generated from patients having adenosine-deaminase deficiency-related severe combined immunodeficiency (ADA-SCID), Duchenne (DMD) and Becker muscular dystrophy (BMD), Parkinson disease (PD) or juvenile-onset type 1 diabetes mellitus (JDM).

14. A method for producing the pseudotyped lentiviral vector particle according to any of claims 1 to 11, the method comprising co-transfecting of a packaging cell line with a psi-negative lentiviral *gaglpol* gene, a psi-positive lentiviral expression vector and one or two psi-negative expression vector(s) encoding for the truncated H and F proteins.

15. A composition comprising the pseudotyped lentiviral vector particle according to any of claims 1 to 11.
